# EUROPEAN PATENT APPLICATION

(11) **EP 3 778 908 A1**
(43) Date of publication of application: **17.02.2021**
(21) Application number: 19774601.9
(22) Date of filing: 20.03.2019
(51) Int. Cl.: C12P 7/06

(54) **METHOD FOR PRODUCING ORGANIC SUBSTANCE**

(30) Priority: 27.03.2018 JP 2018059294
(71) Applicant: SEKISUI CHEMICAL CO., LTD., Osaka-shi Osaka 530-8565 (JP)
(72) Inventor: NISHIYAMA Norihide, Tsukuba-shi, Ibaraki 300-4292 (JP); SATO Kanetomo, Tsukuba-shi, Ibaraki 300-4292 (JP)
(74) Representative: Ter Meer Steinmeister & Partner
(86) International application number: PCT/JP2019/011915
(87) International publication number: WO 2019/188730

(57) **Abstract**

Provided is a method for producing an organic substance, in which an organic material (metabolite) derived from a microorganism is reduced while retaining a nutritive substance in an organic substance-containing solution discharged from a fermentation tank containing the microorganism, whereby various nutritional elements contained in the organic substance-containing solution can be re-used with high efficiency. A method for producing an organic substance by the microbial fermentation of a synthetic gas containing at least carbon monoxide, the method comprising a synthetic gas supply step of supplying the synthetic gas into a fermentation tank containing a microorganism, a fermentation step of subjecting the synthetic gas to microbial fermentation in the fermentation tank, an aerobic fermentation treatment step of subjecting at least a portion of a liquid produced in the fermentation step to an aerobic fermentation treatment in a liquid waste treatment unit, and a recycling step of supplying a liquid produced in the aerobic fermentation treatment step to the fermentation tank, wherein the aerobic fermentation treatment step is carried out in the presence of a nitrifying bacterium inhibitor.

## Description

### Technical Field

The present invention relates to a method for producing an organic substance, and in particular relates to a method for producing an organic substance using a fermented liquid obtained by microbial transformation of synthesis gas.

### Background Art

In recent years, methods for producing various organic substances with a raw material other than petroleum, for example, a method for producing bioethanol from an edible material such as corn by a sugar fermentation method, have been attracting attention, with the concern about depletion of fossil fuel resources and in consideration of global environmental problems such as increase of carbon dioxide in the air.

However, the problem with such sugar fermentation methods using an edible material is that they cause a rise in food prices because the limited farmland area is used for producing materials other than food. To solve this problem, various methods and apparatuses have been studied to produce organic substances using substances as raw materials which have been discarded.

For example, Non Patent Document 1 discloses types of microorganisms which transform synthesis gas containing carbon dioxide, carbon monoxide, and hydrogen into acetic acid and ethanol, and their metabolic system.

Furthermore, Patent Document 1 discloses a method for producing ethanol from exhaust gas from steel mills or synthesis gas and the like obtained by gasification of waste by microbial fermentation.

### Prior Art Documents

### Patent Document

Patent Document 1: JP 2015-53866 A

### Non Patent Document

Non Patent Document 1: Michael Koepke et al., "Clostridium ljungdahlii represents a microbial production platform based on syngas", Proc Natl Acad Sci USA, 2010, vol. 107, 13087-13092

### Summary of Invention

### Technical Problem

The present inventors studied methods for producing organic substances using synthesis gas derived from waste as a raw material by microbial fermentation as disclosed in Patent Document 1, and originally considered a novel method for producing an organic substance effectively by feeding an organic substance-containing solution discharged from a microbial fermenter to the microbial fermenter again.

However, the inventors have found that the organic substance-containing solution is in the form of suspension with an organic matter derived from microorganisms (metabolite), and feeding again the solution directly to the microbial fermenter facilitates contamination in the microbial fermenter, causing a negative impact on culture.

In response to this, the present inventors have discovered that the organic matter derived from microorganisms (metabolite) is successfully decomposed by carrying out an aerobic fermentation treatment before feeding the organic substance-containing solution to the microbial fermenter again, and the contamination in the microbial fermenter is suppressed.

However, the inventors have found that unfavorable nitrifying bacteria also grow in the aerobic fermentation treatment, and thus have faced another problem of transformation of ammonia, which is an important nutrient source for microorganisms, into nitric acid, which cannot be used as a nutrient source for microorganisms, having a negative impact on the production of organic substances.

The present invention has been made in order to solve the above problem, and an object thereof is to provide a method for producing an organic substance efficiently, by decomposing and reducing, by an aerobic fermentation treatment, an organic matter derived from microorganisms (metabolite) in an organic substance-containing solution discharged from a microbial fermenter, and feeding the solution obtained in the aerobic fermentation treatment to the microbial fermenter again, which method can reuse the solution efficiently while retaining nutrients important as a nutrient source for microorganisms, such as ammonia, and can produce an organic substance at high productivity without reducing the activity of microorganisms in the fermenter.

### Solution to Problem

The present inventors have conducted intensive studies on the method for solving the above problem, and have found that an aerobic fermentation treatment of an organic substance-containing solution discharged from a fermenter containing microorganisms in the presence of a nitrifying bacteria inhibitor enables the solution obtained in the aerobic fermentation treatment to be reused efficiently while retaining nutrients important as a nutrient source for microorganisms, such as ammonia, and enables an organic substance to be continuously produced at high productivity without reducing the activity of microorganisms in the fermenter.

Accordingly, the gist of the present invention is as follow:
[1] A method for producing an organic substance by microbial fermentation of synthesis gas containing at least carbon monoxide, the method comprising a synthesis gas feeding step of feeding the synthesis gas to a fermenter containing a microorganism, a fermentation step of carrying out microbial fermentation of the synthesis gas in the fermenter, an aerobic fermentation treatment step of carrying out an aerobic fermentation treatment of at least part of a solution obtained in the fermentation step in a discharge water treatment section, and a recycling step of feeding a solution obtained in the aerobic fermentation treatment step to the fermenter, wherein the aerobic fermentation treatment step is carried out in the presence of a nitrifying bacteria inhibitor.
[2] The method for producing an organic substance according to [1], further comprising a raw material gas-production step of producing a raw material gas by gasification of a carbon source, wherein the synthesis gas comprises the raw material gas.
[3] The method for producing an organic substance according to [1] or [2], wherein the nitrifying bacteria inhibitor is selected from the group consisting of amines such as hydrazine, an isothiazoline compound and dicyandiamide, linoleic acid, α-linolenic acid, γ-linolenic acid, methyl linoleate which is a fatty acid ester, 1-allyl-2-thiourea and 2-chloro-6-(trichloromethyl)pyridine.
[4] The method for producing an organic substance according to any one of [1] to [3], wherein the nitrifying bacteria inhibitor is 1-allyl-2-thiourea.
[5] The method for producing an organic substance according to any one of [1] to [4], wherein a solution subjected to the aerobic fermentation treatment comprises 0.05 to 100 mg/L of the nitrifying bacteria inhibitor.
[6] The method for producing an organic substance according to any one of [1] to [5], further comprising a purification step, wherein the solution subjected to the aerobic fermentation treatment comprises a purification process wastewater obtained in the purification.
[7] The method for producing an organic substance according to any one of [1] to [6], wherein the aerobic fermentation treatment is carried out by an activated sludge method.
[8] The method for producing an organic substance according to any one of [1] to [7], wherein the carbon source comprises waste.
[9] The method for producing an organic substance according to any one of [1] to [8], wherein the fermenter comprises a Clostridium species as the microorganism.
[10] The method for producing an organic substance according to any one of [1] to [9], wherein the organic substance is ethanol.

### Advantageous Effects of Invention

According to the present invention, an organic substance can be produced efficiently by decomposing and reducing, by an aerobic fermentation treatment, an organic matter derived from microorganisms (metabolite) in an organic substance-containing solution discharged from a fermenter containing microorganisms, and feeding the solution obtained in the aerobic fermentation treatment to the fermenter again.

Furthermore, according to the present invention, growth of unfavorable nitrifying bacteria can be suppressed by carrying out an aerobic fermentation treatment of the organic substance discharged from a fermenter containing microorganisms in the presence of a nitrifying bacteria inhibitor. This prevents transformation of ammonia, which is an important nutrient source for microorganisms, into nitric acid, which cannot be used as a nutrient source for microorganisms, and enables the solution obtained in the aerobic fermentation treatment to be reused efficiently while retaining nutrients. As described above, according to the present invention, an organic substance can be efficiently and continuously produced at high productivity without reducing the activity of microorganisms in the fermenter.

### Description of Embodiments

Hereinafter, an example of preferred embodiments of the present invention will be described. The following embodiments are examples for describing the present invention, and the present invention is not at all limited to the following embodiments.

In the present description, the proportion of gas components is based on the volume, not weight, unless otherwise specified. Thus, percentage % represents % by volume and ppm represents ppm by volume unless otherwise specified.

### [I. Substances]

### <Carbon Source>

The carbon source used in the present invention, which is a raw material of synthesis gas, is not particularly limited as long as it produces carbon monoxide or carbon dioxide when burned (gasified). For example, coke in steel mills, coal used in a blast furnace (blast furnace gas), coal used in a convertor or a coal-fired power plant, general waste introduced into an incinerator (in particular, a gasifier), industrial waste and sewage sludge, biomass raw materials, organic compounds including polymer produced as a by-product in various industries and carbon dioxide may be used.

More specifically, carbon sources include household waste in which plastic waste, garbage and household rubbish such as Japanese futon and paper are mixed; industrial waste such as waste tires, plastic waste, food waste and construction waste; forest brushwood such as thinnings; agricultural waste such as bagasse, rice straw, straw and chaff; and fossil fuel such as coal, petroleum, natural gas, shale gas and methane hydrate. While the resulting product is not changed even when the type of carbon sources is different, household waste, industrial waste and municipal solid waste (MSW) including sewage sludge are preferred in consideration of the social goal of reducing the amount of use of natural resources to establish a resource-circulating society. The carbon source is most preferably household waste, which is produced in large quantities and presses disposal sites.

### <Raw material gas>

The raw material gas, which is obtained by gasification of the carbon source, contains carbon monoxide or carbon dioxide as an essential component, and may also contain hydrogen, oxygen and nitrogen. The raw material gas may also contain other components such as soot, tar, a nitrogen compound, a sulfur compound, a phosphorus compound and an aromatic compound.

As described later, the raw material gas may also be produced in the form of a gas containing 20% by volume or more of carbon monoxide, by carrying out a heat treatment (commonly called gasification) in which carbon source is burned (incomplete combustion), in other words, by partly oxidizing carbon source, in a raw material gas-production step.

The above raw material gas-production step includes, for example, burning waste in a gasifier, heating while adding coal in the production of steel (a steel making process), and converting carbon dioxide obtained by burning an organic substance into carbon monoxide by a reverse shift reaction with a metal catalyst.

When the raw material gas is derived from waste, usually the raw material gas is likely to contain 20% by volume or more and 80% by volume or less of carbon monoxide, 10% by volume or more and 40% by volume or less of carbon dioxide, 10% by volume or more and 80% by volume or less of hydrogen, and 1 ppm or more of a nitrogen compound, 1 ppm or more of a sulfur compound, 0.1 ppm or more of a phosphorus compound, and/or 10 ppm or more of an aromatic compound. The raw material gas may also contain other substances such as environmental pollutants, dust particles and impurities. Thus, it is preferable that when feeding synthesis gas to the microbial fermenter, substances unfavorable for stable culture of microorganisms and an unfavorable amount of compounds and the like be reduced or removed from the raw material gas, so that the content of each component in the raw material gas is in the range suitable for stable culture of microorganisms. In particular, many aromatic compounds are cytotoxic, and thus reduction or removal thereof from the raw material gas is preferable.

### <Synthesis gas>

The synthesis gas obtained by gasification of carbon source contains at least carbon monoxide as an essential component, and may further contain hydrogen, carbon dioxide, oxygen, nitrogen, methane and ethane. The synthesis gas may also contain components other than the above components, such as soot, tar, a nitrogen compound, a sulfur compound, a phosphorus compound and an aromatic compound (hereinafter may be referred to as impurities).

The synthesis gas may contain 0.01 ppm or more and 90 ppm or less of at least one compound selected from the group consisting of a sulfur compound, a phosphorus compound and a nitrogen compound. The each content of the above compounds is preferably 0.05 ppm or more, more preferably 0.1 ppm or more, further preferably 0.5 ppm or more, and preferably less than 80 ppm, more preferably 70 ppm or less, further preferably 60 ppm or less, and still more preferably 40 ppm or less. A content ratio of the lower limit or more is advantageous in that microorganisms can be cultured well. A content ratio of the upper limit or less is advantageous in that medium is not contaminated by various nutrient sources which microorganisms have not consumed.

Examples of sulfur compounds usually include sulfur dioxide, CS2, COS, H2S, and in particular H₂S and sulfur dioxide are preferred because they are an easy-to-consume nutrient source for microorganisms. Thus, it is more preferable that the total amount of H₂S and sulfur dioxide in synthesis gas be in the above range, and it is most preferable that the amount of sulfur dioxide alone in synthesis gas be in the above range.

Phosphoric acid is preferred as the phosphorus compound because it is an easy-to-consume nutrient source for microorganisms. Thus, it is more preferable that the synthesis gas contains phosphoric acid in the above range.

Examples of nitrogen compounds include nitrogen monoxide, nitrogen dioxide, acrylonitrile, acetonitrile and HCN. HCN is preferred because it is an easy-to-consume nutrient source for microorganisms. Thus, it is more preferable that the synthesis gas contains HCN in the above range.

The synthesis gas may further contain 0.01 ppm or more and 90 ppm or less of an aromatic compound. The content ratio of the above aromatic compound is preferably 0.03 ppm or more, more preferably 0.05 ppm or more, and further preferably 0.1 ppm or more, and preferably 70 ppm or less, more preferably 50 ppm or less, and further preferably 30 ppm or less. When the content ratio is the lower limit or more, microorganisms are likely to be cultured well. When the content ratio is the upper limit or less, the medium is less likely to be contaminated by various nutrient sources which microorganisms have not consumed. Examples of aromatic compounds include benzene, toluene, ethylbenzene, xylene and styrene. A particularly preferred aromatic compound is xylene.

As described later, as for the synthesis gas, first a raw material gas is produced by gasification of a carbon source (raw material gas production step) and the raw material gas may be directly used; alternatively, a gas obtained through a subsequent step of reducing sulfur concentration in the raw material gas (pretreatment step) may also be used as the synthesis gas. The raw material gas may be prepared in the form of a gas containing carbon monoxide as the main component, by a heat treatment of burning carbon source (incomplete combustion), in other words, by partly oxidizing carbon source. The resulting gas may contain a gas component such as hydrogen, water vapor, carbon dioxide, nitrogen or oxygen in addition to carbon monoxide, and may also contain other components such as soot, tar, a nitrogen compound, a sulfur compound and an aromatic compound.

In this regard, the carbon source to be subjected to the above heat treatment, i.e., gasification, is not particularly limited. For example, various carbon-containing materials such as waste and exhaust gas from steel mills, general waste, and combustion gas from waste incinerators may also be suitably used for the purpose of recycling. For example, as the carbon source, by-products obtained in an industrial process such as manufacture of ferrous products, manufacture of non-ferrous products, petroleum refining process and electric power generation, and modified gas such as biomass gas, natural gas, shale gas and associated petroleum gas may also be used. Utilizing wastes to be discarded as carbon source is important not only economically but also for e consideration for an ecological system. Examples of such waste also include plastic waste, garbage, municipal solid waste (MSW), waste tires, biomass waste, household waste such as Japanese futon and paper and construction members. One of them may be subjected to the gasification treatment singly or two or more of them may be subjected thereto in combination. When the raw material gas is derived from waste, the raw material gas usually contains 1 ppm or more of a nitrogen compound, 100 ppm or more of a sulfur compound, 0.1 ppm or more of a phosphorus compound, and 100 ppm or more of an aromatic compound. Since such waste contains a large amount of, particularly a sulfur compound and an aromatic compound, it is preferable that their content be adjusted to a suitable content in the pretreatment equipment described later before being fed to the continuous fermenter.

As described later, the concentration of carbon dioxide in the synthesis gas is controlled to 0.1% by volume or more, preferably 0.3% by volume or more, more preferably 0.5% by volume or more, further preferably 0.8% by volume or more, and particularly preferably 1% by volume or more, and 9% by volume or less, preferably 8% by volume or less, more preferably 7% by volume or less, further preferably 6% by volume or less, and particularly preferably 5% by volume or less based on the total concentration of carbon monoxide, carbon dioxide, hydrogen and nitrogen in the synthesis gas. The method of controlling the concentration of carbon dioxide in the synthesis gas is not particularly limited as long as it is a known method. For example, the synthesis gas is brought into contact with a PSA (pressure swing adsorption) apparatus packed with an adsorbent capable of adsorbing carbon dioxide, such as zeolite, or a TSA (thermal swing adsorption) apparatus using an amine adsorbing liquid, in the pretreatment step of the raw material gas, to reduce and control the concentration of carbon dioxide in the synthesis gas to the above range.

In the present invention, "control" means that operation is performed in the above condition for 95% or more of the total period from the start of stable production of ethanol to the stop of culture of microorganisms. The initial start-up process of growing an inoculum in the fermenter and the finishing process of stopping feed of medium and synthesis gas to stop culture of the bacteria are not included.

The concentration of carbon monoxide in the synthesis gas is usually 20% by volume or more and 80% by volume or less, preferably 25% by volume or more and 50% by volume or less, and more preferably 35% by volume or more and 45% by volume or less based on the total concentration of carbon monoxide, carbon dioxide, hydrogen and nitrogen in the synthesis gas.

The concentration of hydrogen in the synthesis gas is usually 10% by volume or more and 80% by volume or less, preferably 30% by volume or more and 55% by volume or less, and more preferably 40% by volume or more and 50% by volume or less based on the total concentration of carbon monoxide, carbon dioxide, hydrogen and nitrogen in the synthesis gas.

The concentration of nitrogen in the synthesis gas is usually 40% by volume or less, preferably 1% by volume or more and 20% by volume or less, and more preferably 5% by volume or more and 15% by volume or less based on the total concentration of carbon monoxide, carbon dioxide, hydrogen and nitrogen in the synthesis gas.

The concentration of carbon monoxide, hydrogen and nitrogen may be set at a predetermined range by changing the C-H-N elemental composition of the carbon source or modifying conditions of combustion, such as combustion temperature and the concentration of oxygen in the gas to be fed for combustion in the raw material gas production step. For example, if the concentration of carbon monoxide and hydrogen is to be changed, the carbon source is changed to one with high C/H ratio, such as waste plastic; if the concentration of nitrogen is to be reduced, a gas having high oxygen concentration is fed in the raw material gas production step.

### <Microorganism>

The microorganism (species) for the microbial fermentation of synthesis gas is not particularly limited as long as a desired organic substance can be produced by microbial fermentation of synthesis gas containing carbon monoxide as the main raw material. For example, a microorganism (species) that produces an organic substance from synthesis gas by the fermentation action of gas utilizing bacteria is preferred. Of the gas utilizing bacteria, microorganisms having a COA metabolic circuit which produces an organic substance including acetic acid from carbon monoxide are preferred. The genus Clostridium is more preferred, and Clostridium autoethanogenum is particularly preferred, from the viewpoint of gas utilizing properties and stability of culture. In the following, those microorganisms will be illustrated in more detail.

Gas utilizing bacteria include both eubacteria and archaea.

Examples of eubacteria include bacteria of the genus Clostridium, bacteria of the genus Moorella, bacteria of the genus Acetobacterium, bacteria of the genus Carboxydocella, bacteria of the genus Rhodopseudomonas, bacteria of the genus Eubacterium, bacteria of the genus Butyribacterium, bacteria of the genus Oligotropha, bacteria of the genus Bradyrhizobium, and bacteria of the genus Ralsotonia, which are aerobic hydrogen oxidizing bacteria.

Examples of archaea include bacteria of the genus Methanobacterium, bacteria of the genus Methanobrevibacter, bacteria of the genus Methanocalculus, bacteria of the genus Methanococcus, bacteria of the genus Methanosarcina, bacteria of the genus Methanosphaera, bacteria of the genus Methanothermobacter, bacteria of the genus Methanothrix, bacteria of the genus Methanoculleus, bacteria of the genus Methanofollis, bacteria of the genus Methanogenium, bacteria of the genus Methanospirillium, bacteria of the genus Methanosaeta, bacteria of the genus Thermococcus, bacteria of the genus Thermofilum, and bacteria of the genus Arcaheoglobus. Of them, bacteria of the genus Methanosarcina, bacteria of the genus Methanococcus, bacteria of the genus Methanothermobacter, bacteria of the genus Methanothrix, bacteria of the genus Thermococcus, bacteria of the genus Thermofilum, and bacteria of the genus Archaeoglobus are preferred as archaea.

Furthermore, bacteria of the genus Methanosarcina, bacteria of the genus Methanothermobactor, or bacteria of the genus Methanococcus are preferred, and bacteria of the genus Methanosarcina or bacteria of the genus Methanococcus are more preferred as archaea because they have excellent properties of utilizing carbon monoxide and carbon dioxide. Specific examples of bacteria of the genus Methanosarcina include Methanosarcina barkeri, Methanosarcina mazei and Methanosarcina acetivorans.

Of the above gas utilizing bacteria, a bacterium which is highly capable of producing the target organic substance is selected and used. Examples of gas utilizing bacteria highly capable of producing ethanol include Clostridium autoethanogenum, Clostridium ljungdahlii, Clostridium aceticum, Clostridium carboxidivorans, Moorella thermoacetica and Acetobacterium woodii.

### <Medium>

In the present invention, the medium used for culturing the above microorganisms is not particularly limited as long as it has a composition suitable for the bacteria.

### <Organic substance and its application>

The organic substance produced by the method of the present invention is not particularly limited as long as it is an organic substance produced from carbon monoxide by microbial fermentation. Examples thereof include compounds such as alcohol having 1 to 12 carbon atoms, diol having 1 to 12 carbon atoms, acetic acid, lactic acid, isoprene and butadiene. The organic substance is preferably alcohol or diol having 1 to 4 carbon atoms, and more preferably ethanol. These organic substances may be used as a raw material for plastics and resins, and also as various solvents, disinfectants or fuels. High concentration of ethanol has a wide range of applications, and can be used as fuel ethanol to be mixed to gasoline, a raw material for cosmetics, beverage, chemical substances and fuel (jet fuel), and an additive for food.

### [II. Method for production]

### <Raw material gas production step>

The raw material gas production step (gasification step) is a step of producing the above raw material gas by gasification of the above carbon source. In the raw material gas production step, a gasifier may be used. The gasifier is a furnace for burning carbon source (incomplete combustion). Examples thereof include a shaft furnace, a kiln furnace, a fluidized bed furnace and a gasification reforming furnace. It is preferable that the gasifier is a fluidized bed furnace because high heating rate in the hearth and excellent operability are achieved when waste is partly burned, but the gasifier is not limited thereto. High heating rate in the hearth and excellent operability are achieved when waste such as rubbish is partly burned. Gasification may be performed by heating (baking) waste in a low oxygen atmosphere at, for example, 450 to 600°C and thermally decomposing it into gas (carbon monoxide, carbon dioxide, hydrogen, methane and the like) and char which contains a large amount of carbon. As described above, when gasification is performed in a fluidized bed furnace in an atmosphere at relatively low temperature and low oxygen, incombustibles in waste can be separated more hygienically and with lower oxidation degree than in the hearth, and thus valuables in incombustible matters such as aluminum and iron can be selected and recovered. Thus, utilizing such a process to achieve an efficient recycling of resources is another industrial advantage.

The temperature of the above gasification in the raw material gas production step is usually 100°C or more and 1500°C or less, and preferably 200°C or more and 1200°C or less.

The reaction time of the gasification in the raw material gas production step is usually 2 seconds or more, and preferably 5 seconds or more.

### <Pretreatment step>

If the composition of various gases and the amount of impurities in the synthesis gas are not suitable for culturing microorganisms, a pretreatment step may be added.

The pretreatment step is a step of removing and reducing components in the synthesis gas not suitable for the culture of microorganisms to give a purified gas having a composition suitable for microbial fermentation.

The above unsuitable components mean a component not suitable for microorganisms, and examples thereof include the above cytotoxic impurities; components interfering with apparatuses in the pretreatment step, and gas components poisonous to anaerobic microorganisms, such as oxygen.

These cytotoxic impurities, components interfering with apparatuses and poisonous gas components can be suitably selected depending on the apparatus and the species of microorganisms selected.

The pretreatment step may be performed by using one or two or more apparatuses of, for example, a scrubber (separator for water-soluble impurities), a gas chiller (water separator), a separator for fine particles (soot) such as a cyclone and a bag filter, a desulfurizer (separator for sulfide), a separator with a low temperature separation system (cryogenic system), a separator with a membrane separation system, a separator with a pressure swing adsorption system (PSA), a separator with a temperature swing adsorption system (TSA), a separator with a pressure and temperature swing adsorption system (PTSA), a deoxidization apparatus, a separator using activated carbon and a separator using a copper catalyst or a palladium catalyst. In the present invention, the pretreatment step may be a step of treating a raw material gas by a scrubber and/or an adsorption apparatus.

The scrubber is used to remove pollutants and the like in gas. Any one of wet cleaning methods or dry cleaning methods may be used depending on the purpose. Of them, a wet cleaning method in which a particulate substance is brought into contact with a cleaning solution may be preferably used. An example thereof to be used is a cleaning method using what is called water curtain. When the wet cleaning method is used, water, an acidic solution, an alkaline solution and the like may be used as a cleaning solution. Of them, water is preferred. Furthermore, the temperature of the cleaning solution is usually 40°C or less, preferably 30°C or less, more preferably 25°C or less, and most preferably 15°C or less. The scrubber is preferably used to remove soot, tar and an aromatic compound.

An adsorption apparatus capable of adsorbing carbon monoxide in gas may be used. Examples of units exclusively for adsorption include a desulfurization layer and a deoxidization layer.

Of them, the desulfurization layer is not particularly limited as long as it can remove sulfur components. If sulfur components are not thoroughly removed or reduced by the desulfurization layer and remain in a large amount, this may have a negative impact on an adsorption/desorption unit when there is such a unit at the subsequent stage.

Meanwhile, the deoxidization layer is not particularly limited as long as it can remove oxygen components. When oxygen components are not thoroughly removed or reduced by the deoxidization layer and remain in a large amount, microorganisms used in the present invention, in particular, anaerobic microorganisms may be killed.

Furthermore, an adsorption/desorption apparatus may be provided, and in such cases, any one of a PSA, a TSA and a PTSA may be preferably used. Any other units may be arbitrarily provided to further remove undesired impurities.

Porous materials such as activated carbon, zeolite and molecular sieves and an aqueous solution such as an amine solution may be used as an adsorbing/desorbing material used in the adsorption/desorption apparatus. Of them, activated carbon or zeolite, which can adsorb aromatic compounds and sulfur compounds, are preferably used. As described above, since a high sulfur content and a high water content of gas may have a negative impact on the adsorbing/desorbing material, it is preferable that the adsorption/desorption apparatus be installed downstream of the desulfurization layer.

### <Synthesis gas feeding step>

The synthesis gas feeding step is a step of feeding the synthesis gas obtained as described above to a fermenter containing microorganisms. The synthesis gas to be fed may contain hydrogen in addition to carbon monoxide, and the characteristic is that the content of carbon dioxide is controlled to 0.1% by volume or more and 9% by volume or less based on the total concentration of carbon monoxide, carbon dioxide, hydrogen and nitrogen in the synthesis gas. In the present invention, setting the amount of carbon dioxide in the synthesis gas to the above range enables carbon monoxide in the synthesis gas to be converted into an organic substance highly efficiently. Although the reason is not clear, it can be assumed as follows.

When an organic substance is prepared by microbial fermentation of synthesis gas containing carbon monoxide, carbon dioxide and hydrogen, microorganisms (e.g., ethanol-producing bacteria) are considered to produce ethanol through acetic acid as disclosed in Non-Patent Document 1 and the like. At that stage, ethanol is produced from not only carbon monoxide but also carbon dioxide through acetic acid as shown in Figure 1. Thus, attention has not been paid to carbon dioxide in synthesis gas. For example, while a raw material gas obtained after gasification of exhaust gas from steel mills or carbon source is known to contain about 10 to 30% by volume of carbon dioxide, it has been thought that the concentration of carbon dioxide in the raw material gas needs not be controlled when subjecting the raw material gas to microbial fermentation for the above reason.

However, the energy level of carbon monoxide is different from that of carbon dioxide, and since the energy level of carbon dioxide is lower than that of carbon monoxide, and carbon dioxide is stable, the energy necessary for producing ethanol from carbon dioxide through acetic acid is considered to be higher than that for producing ethanol from carbon monoxide through acetic acid. Then, a higher concentration of carbon dioxide increases the amount of acetic acid produced as a by-product, reducing the amount of ethanol to be produced. Thus, in the present invention, the upper limit of the concentration of carbon dioxide in synthesis gas is 9% by volume, preferably 8% by volume or less, more preferably 7% by volume or less, further preferably 6% by volume or less, and particularly preferably 5% by volume or less.

Furthermore, if the synthesis gas to be subjected to microbial fermentation contains no carbon dioxide (in other words, if the concentration of carbon dioxide is 0%), the amount of ethanol to be produced is reduced. It has been believed that in the process for producing acetic acid or ethanol from carbon monoxide by microbial fermentation, microorganisms take carbon monoxide and convert it into carbon dioxide as shown in Figure 1, and thus intentional feeding of carbon dioxide to microorganisms from outside is unnecessary. The present inventors have found that using synthesis gas containing a small amount of carbon dioxide improves productivity of ethanol. This may be because a small amount of carbon dioxide in synthesis gas improves the balance of the metabolic system of microorganisms, and consequently improves productivity of ethanol. For this reason, in the present invention, the lower limit of the concentration of carbon dioxide in synthesis gas is 0.1% by volume, preferably 0.3% by volume or more, more preferably 0.5% by volume or more, further preferably 0.8% by volume or more, and particularly preferably 1% by volume or more.

As long as the condition of the component of synthesis gas is met, a gas obtained through the raw material gas production step and the subsequent pretreatment step may be directly used as synthesis gas. Alternatively, another predetermined gas may be added to the gas obtained through the raw material gas production step and the pretreatment step to use it as synthesis gas. For example, at least one compound selected from the group consisting of a sulfur compound such as sulfur dioxide, a phosphorus compound and a nitrogen compound may be added as another predetermined gas to form synthesis gas.

### <Fermentation step>

The fermentation step according to the present invention is a step of microbial fermentation of the above synthesis gas to produce an organic substance. A fermenter containing the above microorganisms (species) is used in the fermentation step. The fermenter may contain a medium (culture solution) in addition to microbial species. Some anaerobic microorganisms are known to produce an organic substance, which is a valuable such as ethanol, from substrate gas such as synthesis gas by their fermentation activity. These types of gas utilizing bacteria are cultured in a liquid medium. For example, a culture solution and gas utilizing bacteria are fed to and stored in a fermenter, and with stirring the culture solution in that state, synthesis gas may be fed to the fermenter. This allows gas utilizing bacteria to be cultured in the culture solution and an organic substance can be produced from synthesis gas by the fermentation action.

The culture solution contains water, the main component, and nutrients dissolved or dispersed in water (e.g., vitamins, phosphoric acid). The composition of such a culture solution is adjusted so that gas utilizing bacteria grow well.

It is preferable that the fermenter is a continuous fermentation apparatus. A microbial fermenter of any form may be usually used. Examples thereof include a stirring type, an airlift type, a bubble column type, a loop type, an open pond type, and a photobioreactor type.

### <Purification step>

In the method for producing an organic substance of the present invention, the organic substance-containing solution obtained by microbial fermentation in the fermentation step may be then subjected to a purification step for the purpose of purification. The purification step is a step for purifying (concentrating) the desired organic substance from the organic substance-containing solution obtained in the fermentation step. A step of solid-liquid separation of separating microorganisms from the organic substance-containing solution previously may be included depending on the method of purification described later. Examples of apparatus used in the step of solid-liquid separation include a ceramic filter, a centrifugation apparatus and a sand filtration apparatus. An appropriate apparatus may be selected depending on the particle size of microorganisms.

Examples of apparatus used in the purification step include a distiller, a treatment apparatus including a pervaporation membrane, a treatment apparatus including a zeolite dehydration membrane, a treatment apparatus for removing a low boiling point substance whose boiling point is lower than that of an organic substance, a treatment apparatus for removing a high boiling point substance whose boiling point is higher than that of an organic substance, and a treatment apparatus including an ion exchange membrane. These apparatuses may be used singly or in combination of two or more. For unit operation, heating distillation and membrane separation may be suitably used.

In heating distillation, a distiller may be used to purify the desired organic substance at high purity. The temperature in the distiller during distillation of an organic substance (particularly ethanol) is not particularly limited, and is preferably 100°C or less, and more preferably about 70 to 95°C. Setting the temperature in the distiller to the above range ensures separation of the necessary organic substance from other components, i.e., distillation (purification) of the organic substance.

The pressure in the distiller in distillation of organic substances may be normal pressure, preferably less than atmospheric pressure, and more preferably about 60 to 95 kPa (gauge pressure). Setting the pressure in the distiller to the above range can improve the efficiency of separation of organic substances and thus can improve the yield of organic substances. The yield of the organic substance (concentration of the organic substance obtained after distillation) is preferably 90% by weight or more, more preferably 99% by weight or more, and particularly preferably 99.5% by weight or more.

In the membrane separation, a membrane most suitable for the intended organic substance may be selected from known water-separation membranes such as a zeolite membrane, a dendrimer membrane and a hollow fiber membrane.

The wastewater (purification process wastewater) obtained in the purification step may be used in the aerobic fermentation treatment step described later from the viewpoint of reuse. For example, the wastewater may be mixed to the solution to be treated by aerobic fermentation. In an example, when at least part of the solution obtained in the fermentation step (organic substance-containing solution) is subjected to the aerobic fermentation treatment in a wastewater treatment section, the purification process wastewater may be added to the part of the solution. Reusing the purification process wastewater in that way leads to suppression of manufacturing cost and reduction of the amount of the purification process wastewater to be discarded, and thus can be environmentally friendly.

### <Aerobic fermentation treatment step>

It is preferable that the method for producing an organic substance of the present invention include an aerobic fermentation treatment step, in which at least part of the solution containing an organic substance (organic substance-containing solution) obtained by microbial fermentation in the fermentation step is subjected to an aerobic fermentation treatment in a discharge water treatment apparatus such as a discharge water treatment section.

It is preferable that the aerobic fermentation treatment step is carried out in the presence of a nitrifying bacteria inhibitor.

Growth of unfavorable nitrifying bacteria can be suppressed by carrying out an aerobic fermentation treatment of the organic substance discharged from a fermenter containing microorganisms in the presence of a nitrifying bacteria inhibitor as described above. This prevents transformation of ammonia, which is an important nutrient source for microorganisms, into nitric acid, which cannot be used as a nutrient source for microorganisms, and enables the solution obtained in the aerobic fermentation treatment to be reused efficiently while retaining nutrients. As described above, by using the method of the present invention, an organic substance can be efficiently and continuously produced at high productivity without reducing the activity of microorganisms in the fermenter.

Examples of nitrifying bacteria inhibitors include inhibitors whose target is ammonia monooxygenase (AMO) and hydroxylamine oxidoreductase (HAO). In particular, a nitrifying bacteria inhibitor selected from the group consisting of amines such as hydrazine, an isothiazoline compound and dicyandiamide, linoleic acid, α-linolenic acid, γ-linolenic acid, methyl linoleate which is a fatty acid ester, 1-allyl-2-thiourea and 2-chloro-6-(trichloromethyl)pyridine may be suitably used. Of them, 1-allyl-2-thiourea or 2-chloro-6-(trichloromethyl)pyridine is preferred because of their low volatility, and 1-allyl-2-thiourea is most preferred. Using such a specific nitrifying bacteria inhibitor is particularly preferred because a sufficient effect of inhibiting nitrifying bacteria can be obtained without affecting the fermentation step.

In an embodiment of the present invention, the solution to be treated by aerobic fermentation contains 0.05 mg/L or more, preferably 0.1 mg/L or more, more preferably 1 mg/L or more, and 100 mg/L or less, preferably 10 mg/L or less, more preferably 1 mg/L or less of a nitrifying bacteria inhibitor. Containing a nitrifying bacteria inhibitor in an amount of the lower limit or more as described above ensures a sufficient effect of inhibiting nitrifying bacteria. Furthermore, containing a nitrifying bacteria inhibitor in an amount of the upper limit or less sufficiently suppresses adverse effects on microorganisms, making it possible to maintain the activity of microorganisms.

The aerobic fermentation treatment step is not particularly limited as long as the object of the present invention is achieved. It is preferable that the step is performed by an activated sludge method. The activated sludge method uses activated sludge containing a large amount of microorganisms such as bacteria, protozoa and metazoa to treat a liquid of interest. Contaminants in the liquid, which are mainly composed of organic substances, are decomposed and absorbed by microorganisms in the activated sludge, and thus are removed from the liquid of interest. Another example of activated sludge methods include a method in which oxygen is fed to the liquid to be treated to maintain aerobic conditions, and oil and fat components are decomposed and utilized by using the metabolic action of aerobic microorganisms. Use of such an activated sludge method can simplify the process of treatment and can suppress the running cost.

### <Recycling step>

It is preferable that the method for producing an organic substance of the present invention includes a recycling step of feeding a solution containing an organic substance (organic substance-containing solution) obtained through the aerobic fermentation treatment step to the fermenter containing microorganisms again. As described above, by using the method of the present invention, an increase in the manufacturing cost can be suppressed and an organic substance can be efficiently and continuously produced at high productivity without reducing the activity of microorganisms in the fermenter.

### <Organic substance and its application>

Examples of organic substances produced by the method of the present invention include methanol, ethanol, 2,3-butanediol, acetic acid, lactic acid, isoprene and butadiene. The organic substance is preferably an alcohol or a diol having 1 to 4 carbon atoms, and more preferably contains ethanol. The application of the organic substance produced by the method of the present invention is not particularly limited. The organic substance produced may be used as a raw material for plastics and resins, and also as various solvents, disinfectants or fuels. High concentration ethanol has a wide range of applications, and can be used as fuel ethanol to be mixed into gasoline, a raw material for cosmetics, beverage, chemical substances and fuel (jet fuel), and an additive for food.

### [III. Action of present invention]

Examples described later have demonstrated that a desired organic substance was suitably produced at high productivity by using the method of the present invention. The action of the present invention including details of studies is assumed to be as follows.

Studies by the present inventors have revealed the problem of generation of nitrifying bacteria as a contaminant and their growth in the microbial group when going through the aerobic fermentation treatment step of bringing an organic substance-containing solution into contact with activated sludge. Usually a large amount of air is used as an oxygen source in the aerobic fermentation treatment step, and thus one reason is the presence of a substance in the air, which can be a contamination source. However, it is actually very difficult to block all of such contamination sources.

In such circumstance, the present inventors have originally conceived of carrying out an aerobic fermentation treatment step in the presence of a nitrifying bacteria inhibitor and have embodied the idea through experiment, and as a result, unfavorable nitrifying bacteria were successfully killed or their growth was actually inhibited, and it is consequently considered that the above problem has been solved.

Furthermore, according to the studies of the present inventors, it was necessary to find a nitrifying bacteria inhibitor having a sufficient effect of inhibiting nitrifying bacteria and having no negative impact on the culture in a microbial fermenter, when carrying out experiment of feeding a solution of interest containing a nitrifying bacteria inhibitor to a fermenter containing microorganisms again. As a result of the present inventors' intensive studies, they have conceived of using a nitrifying bacteria inhibitor having a specific structure out of a wide variety of disinfectants, and they have embodied and verified the idea through experiment and it is considered that the above problem has been successfully solved.

### Examples

The present invention will be described in more detail with reference to the following Examples, but the present invention is not limited to the following Examples.

### [Example 1]

A continuous fermentation apparatus (fermenter) equipped with a main reactor, a synthesis gas feeding port, a medium feeding port and a discharge port was filled with an inoculum of Clostridium autoethanogenum (a microorganism) and a liquid medium for culturing the bacterium (containing a suitable amount of a phosphorous compound, a nitrogen compound and various minerals).

Next, synthesis gas composed of 30% by volume of carbon monoxide, 10% by volume of carbon dioxide, 35% by volume of hydrogen and 25% by volume of nitrogen was prepared and fed to the continuous fermentation apparatus to perform culture (microbial fermentation).

After the culture, an ethanol-containing solution discharged from the microbial fermenter was collected, and the solution was continuously boiled in a distillation tower and separated into a purified ethanol solution and purification process discharge water. Next, a nitrifying bacteria inhibitor, 1-allyl-2-thiourea, was added to the purification process discharge water at 2 mg/L, and subsequently the biochemical oxygen demand in the condition of inhibiting nitrifying bacteria (hereinafter may be referred to as "ATU-BOD") was measured according to the method of JIS K0102.

ATU-BOD is a method of measurement known to those skilled in the art, and refers to the biochemical oxygen demand (BOD) when nitrifying action is inhibited by adding N-allylthiourea (ATU) to a liquid sample. Use of ATU-BOD is advantageous in that the influence by the amount of nitrifying bacteria is reduced and the amount of oxygen consumed only by an organic substance can be measured.

In Example 1, the experiment in the same conditions was carried out on different days for a total of 3 times. The results of measurement of ATU-BOD are shown in Table 1.

### [Comparative Example 1]

Experiment was carried out in the same manner as in Example 1 except that the nitrifying bacteria inhibitor was not added. In other words, the biochemical oxygen demand in the coexistence of nitrifying bacteria (hereinafter may be referred to as "BOD") was measured.

The experiment in the same conditions was carried out on different days for a total of 3 times also in Comparative Example 1. The results of measurement of BOD are shown in Table 1.

**[Table 1]**

| | Biochemical oxygen demand | First | Second | Third |
|---|---|---|---|---|
| Example 1 (nitrifying bacteria inhibitor added) | ATU-BOD (mg/L) | 10, 000 | 10, 000 | 11, 000 |
| Comparative Example 1 (nitrifying bacteria inhibitor not added) | BOD (mg/L) | 11, 000 | 11, 000 | 12, 000 |

### <Discussion>

As described in Table 1, a comparison of the results of the above multiple experiments shows that values of biochemical oxygen demand in Example 1 are smaller than those in Comparative Example 1, indicating a clear difference between the two. In other words, in Example 1, the presence of the nitrifying bacteria inhibitor, 1-allyl-2-thiourea, suppressed the activity of unfavorable nitrifying bacteria, and as a result, it is confirmed that the values of biochemical oxygen demand are smaller than those in Comparative Example.

### [Example 2]

A continuous fermentation apparatus (fermenter) equipped with a main reactor, a synthesis gas feeding port, a medium feeding port and a discharge port was filled with an inoculum of Clostridium autoethanogenum (a microorganism) and a liquid medium for culturing the bacterium (containing a suitable amount of a phosphorous compound, a nitrogen compound and various minerals).

Next, synthesis gas composed of 30% by volume of carbon monoxide, 10% by volume of carbon dioxide, 35% by volume of hydrogen and 25% by volume of nitrogen was prepared and fed to the continuous fermentation apparatus to perform culture (microbial fermentation).

The progress was observed for 25 days from the start of culture. The synthesis gas was continuously utilized to give ethanol in a stable manner.

A nitrifying bacteria inhibitor, 1-allyl-2-thiourea, was added to the fermenter 25 days after the start of the culture so that the concentration was 2 mg/L based on the total amount of the solution in the fermenter.

Then the progress was observed for 24 hours and conversion of synthesis gas into ethanol was measured, and no significant change was found.

### <Discussion>

The above results have revealed that the nitrifying bacteria inhibitor, 1-allyl-2-thiourea, used in Examples 1 and 2 has no negative impact on microorganisms in the fermenter and is a suitable nitrifying bacteria inhibitor in the production of ethanol, which is an organic substance.

The method for producing an organic substance of the present invention has been illustrated non-limitedly with reference to preferred embodiments of the present invention above. Those skilled in the art should understand that modification and/or revision may be made without departing from the scope of the invention defined in the appended claims.

## Claims

1. A method for producing an organic substance by microbial fermentation of synthesis gas containing at least carbon monoxide, the method comprising:
a synthesis gas feeding step of feeding the synthesis gas to a fermenter containing a microorganism;
a fermentation step of carrying out microbial fermentation of the synthesis gas in the fermenter;
an aerobic fermentation treatment step of carrying out aerobic fermentation treatment of at least part of a solution obtained in the fermentation step in a discharge water treatment section; and
a recycling step of feeding a solution obtained in the aerobic fermentation treatment step to the fermenter,
wherein the aerobic fermentation treatment step is carried out in the presence of a nitrifying bacteria inhibitor.

2. The method for producing an organic substance according to Claim 1, further comprising a raw material gas-production step of producing a raw material gas by gasification of a carbon source, wherein the synthesis gas comprises the raw material gas.

3. The method for producing an organic substance according to Claim 1 or 2,
wherein the nitrifying bacteria inhibitor is selected from the group consisting of amines such as hydrazine, an isothiazoline compound and dicyandiamide, linoleic acid, α-linolenic acid, γ-linolenic acid, methyl linoleate which is a fatty acid ester, 1-allyl-2-thiourea and 2-chloro-6-(trichloromethyl)pyridine.

4. The method for producing an organic substance according to any one of Claims 1 to 3, wherein the nitrifying bacteria inhibitor is 1-allyl-2-thiourea.

5. The method for producing an organic substance according to any one of Claims 1 to 4, wherein a solution subjected to the aerobic fermentation treatment comprises 0.05 to 100 mg/L of the nitrifying bacteria inhibitor.

6. The method for producing an organic substance according to any one of Claims 1 to 5, further comprising a purification step, wherein the solution subjected to the aerobic fermentation treatment comprises a purification process wastewater obtained in the purification.

7. The method for producing an organic substance according to any one of Claims 1 to 6, wherein the aerobic fermentation treatment is carried out by an activated sludge method.

8. The method for producing an organic substance according to any one of Claims 1 to 7, wherein the carbon source comprises waste.

9. The method for producing an organic substance according to any one of Claims 1 to 8, wherein the fermenter comprises a Clostridium species as the microorganism.

10. The method for producing an organic substance according to any one of Claims 1 to 9, wherein the organic substance is ethanol.
